# EUROPEAN PATENT SPECIFICATION

(11) **EP 2 247 204 B1**
(45) Date of publication and mention of the grant of the patent: **03.07.2019**
(21) Application number: 09708295.2
(22) Date of filing: 29.01.2009
(51) Int. Cl.: C13K 1/06, A23L 2/52, A23L 2/62, C08B 31/00, C12P 19/04, C12P 19/14, A23L 29/219

(54) **PREPARATION OF ENZYMATICALLY HYDROLYSED STARCH**
HERSTELLUNG VON ENZYMATISCH HYDROLYSIERTER STÄRKE
PRÉPARATION D'AMIDON HYDROLYSÉ PAR VOIE ENZYMATIQUE

(30) Priority: 30.01.2008 US 6295808 P
(43) Date of publication of application: 10.11.2010
(73) Proprietor: Cargill, Incorporated, Wayzata, MN 55391 (US)
(72) Inventor: FONTEYN, Dirk, B-2820 Bonheiden (BE); HEIGIS, John R., Cedar Rapids Iowa 52403 (US); HOLTWICK, Joseph B., Cedar Rapids Iowa 52402 (US); MAURO, David J., Dolton Illinois 60419 (US); HOMSMA, Catharina H., B-3060 Bertem (BE); PROVOOST, Dirk R., B-1800 Vilvoorde (BE); SHIEH, Wen-Juin, Munster Indiana 46321 (US); STEPHENS, Blair C., Dayton Ohio 45414 (US)
(74) Representative: Elseviers, Myriam
(86) International application number: PCT/US2009/000570
(87) International publication number: WO 2009/099546

(56) References cited:
- EP-A1- 0 332 027
- US-A- 5 089 171
- US-A- 6 096 524
- US-A- 6 096 524
- US-A1- 2002 142 087
- US-A1- 2006 060 814
- TESCH S ET AL: "STABILIZATION OF EMULSIONS BY OSA STARCHES", JOURNAL OF FOOD ENGINEERING, BARKING, ESSEX, GB, vol. 54, no. 2, 1 January 2002 (2002-01-01), pages 167-174, XP001184187, ISSN: 0260-8774, DOI: 10.1016/S0260-8774(01)00206-0
- MARC J E C VAN DER MAAREL ET AL: "Properties and applications of starch-converting enzymes of the alpha-amylase family", JOURNAL OF BIOTECHNOLOGY, ELSEVIER, AMSTERDAM, NL, vol. 94, no. 2, 1 January 2002 (2002-01-01), pages 137-155, XP002270826, ISSN: 0168-1656, DOI: 10.1016/S0168-1656(01)00407-2

## Description

### FIELD OF THE INVENTION

The present invention relates to methods for preparing enzymatically hydrolyzed starch for use as a stabilizing agent. The present invention also relates to emulsions and food products containing such enzymatically hydrolyzed starch.

### BACKGROUND OF THE INVENTION

Chemical compositions such as guar gum, gum Arabic, and other gums, starches, proteins, various water soluble polymers, and the like are often used as emulsifying and stabilizing agents in food, cosmetic, pharmaceutical and various industrial applications. Gum Arabic is frequently selected for its superior shelf stability, long history of use, natural perception by consumers, and ease of use, particularly during refrigerated or frozen storage of the emulsion. Gum Arabic is expensive, however, and its supply and quality are unpredictable. In addition, it is also often necessary to use gum Arabic at relatively high levels in formulations to meet functional performance requirements. Thus, industry has long searched for a shelf stable, low cost replacement for gum Arabic. Starch derived products have been suggested for such use.

US 6 096 524 discloses a starch ester having a degree of substitution between 1.0 and 1.8, which is useful in manufacturing aqueous emulsions. EP 0 332 027 discloses a method using enzymatic degradation for preparing a modified starch, e.g. a nOSA starch, having emulsification properties. US 2002/142087 also discloses a method of making a nOSA starch by enzymatic modification. TECH S ET AL "STABILIZATION OF EMULSION BY OSA STARCHES", Journal of Food Engineering, Barking, Essex, GB, vol. 54, no. 2, 1 January 2002(2002-01-01, pages 167-174, is a study of the stabilization of emulsions by nOSA starches.

A drawback to the use of the known starch derived products in replacing gum Arabic, however, is that known starch derivatives are less stable during storage. These starch derivatives display shorter shelf life and poor refrigeration and freeze/thaw stability compared to gum Arabic. Starch derivatives are susceptible to being hydrolyzed with acids or enzymes in a random, non-selective pattern that produces starch fragments that have little capacity to emulsify.

The stability problem in beverage applications is thought to occur for a variety of reasons. While not intending to be bound by theory, a description of the current understanding in beverage emulsion stabilization is useful to illustrate why a combination of requirements is typically necessary to provide a suitable beverage emulsifier. A beverage emulsion is often a mechanically emulsified mixture of immiscible solutions of polar and non-polar liquids. The gum or starch is mixed with water to form a solution or suspension of molecules or small particles dispersed in the liquid medium. A flavor oil or other nonaqueous ingredient is added and mechanical agitation is imposed. Often the emulsion is created in two steps, first with relatively low energy agitation to make a coarse emulsion and then second with high-pressure homogenization to make a fine emulsion. Whether a fine or coarse emulsion results from the mechanical energy imposed on the mixture, whether the emulsion is stable over time, and whether the mixture is an oil in water or water in oil emulsion, can be influenced by the viscosity of the oil and the water phases in addition to other aspects discussed later. The viscosity of the oil phase is not something often modified by additional ingredients although the oil itself can be modified or selected to have a certain viscosity at a known temperature and shear rate. The temperature of the oil phase can be used to modify the viscosity during homogenization. As the temperature increases the oil viscosity typically decreases. The viscosity of the water phase is governed by the concentration and molecular weight and molecular architecture of the beverage emulsion stabilizer. The difference between the water phase viscosity and the oil phase viscosity must be matched with the mechanical energy and the temperature during emulsifying to finally perform in the finished beverage concentrate emulsion. The tendency of the mechanically formed emulsion, due to the forces such as electrostatic repulsion, surface tension, density differences causing fluid motion, Brownian motion, and osmotic pressures causing depletion flocculation, generally favor separation of the emulsion into an oil and water phase. Droplets of oil in the oil-in-water emulsion can coalesce, flocculate, cream, sediment or change in size, any of which will create a failure in the application. The stability of an emulsion can therefore be governed by attributes such as continuous phase and discrete phase viscosity, surface active agents present in the formulation, size and size distribution of the oil droplets, density differences between phases, storage conditions, and other ingredient interactions.

Beverage emulsion stabilizers can act to decrease the forces that tend to destabilize an emulsion. One generally accepted theory holds that a molecule with hydrophobic and hydrophilic ends can stabilize the surface and preserve the separation at the interface between the non-polar and polar surface. Soap bubbles are thought to act in this way by lining up in a linear fashion at the interface with the non-polar end pointed into the oil phase and the polar head pointed into the water phase. The critical micelle concentration occurs when there are enough molecules of high enough molecular weight and polar/non-polar charge to raise the surface tension above the forces acting to destabilize the surface. As the discrete oil phase droplet size is decreased with higher energy input during homogenization, the total oil-water phase interfacial surface area increases. Thus, as the oil droplet size decreases, either a higher micelle concentration or higher activity emulsion stabilizer should be used. With larger molecules, such as starch or gums, the interfacial surface is thought to be further stabilized by the bridging action of molecular entanglement of the polymer chains in the polar water phase of the emulsion. The critical micelle concentration wherein the emulsion is stable to coalescence is therefore decreased with increasing entanglement of the polymer chains. Lower concentrations of the emulsion stabilizer will effectively preserve the discrete phase intact. In the oil phase it is generally understood that, as the molecular weight of the hydrophobic end groups is raised from single hydrocarbon to multiple carbon chains, the capacity to interact with the oil becomes stronger. Gum Arabic is known to contain many charged groups, which have a strong interaction with both the oil phase and the water phase. Starch substituted with the food grade octenyl succinic anhydride is also known to have higher capacity to stabilize oil in water emulsions compared to starch which does not have a hydrophobic moiety attached. When the hydrophobic group is sterically hindered in its exposure to the oil phase, its capacity to interact and stabilize is reduced. When the hydrophobic group is located on the exterior of the molecule and fully open to interact with the oil phase, the capacity to stabilize the emulsion is increased. Enzymes can be used to cleave down to the active group, or chemical derivitization can be done in a locationally selective manner to add non-homogeneously and cause higher concentration of substituents on the exterior of the emulsifier.

In addition to the previously described viscosity issues, currently available starch products have a tendency to retrograde, which cause, for example, break down of the flavor oil emulsion upon temperature cycling or long-term storage. Retrogradation has been partially overcome in certain applications by chemically derivitizing the starch molecule to stabilize the starch. These modifications interfere with the association between starch molecules, or portions of the same molecule, and thereby reducing the tendency of the starch to lose its hydration ability on storage. For example, reacting the starch with a reagent to introduce substituents such as hydroxypropyl, phosphate, and acetate or succinate groups tends to stabilize the starch molecule during storage. These reactions may be carried out on starches, which are further modified by crosslinking or degradation to obtain starches for particular applications. Still, these starches do not provide the stable emulsification properties typical of gum Arabic.

Other processes treat the starch with an exo-enzyme, such as a beta-amylase, which cleaves maltose from the non-reducing end of the polymer. While the resulting, modified starch derivatives exhibit a reduced tendency to retrograde during storage, beta-amylase of suitable purity, without alpha-amylase contamination is not readily available and is expensive to use in manufacturing the products. The alpha-amylase contamination reacts with the starch and reduces the viscosity below the level at which the starch is functional. The beta-amylase must therefore be extensively tested and specifically selected to find commercial batches which are low in alpha-amylase contamination and can produce starch of sufficient viscosity to stabilize an emulsion or perform in other applications. Even with this selection, the starch products hydrolyzed with beta-amylase exhibit a low viscosity that is often not sufficient to kinematically stabilize emulsions. The limit dextrin molecules produced by beta-amylase are relatively functional emulsifiers, by virtue of the selective non-reducing end mode of hydrolysis of the enzyme, which theoretically exposes the active hydrophobic moiety to the oil droplet more readily than with a molecule randomly cleaved. The beta-amylase enzyme will only cleave consecutive maltose units from the end of the starch chain until a chemical substituent or a branch point in the starch is reached. The maltose byproduct of beta-amylase that often represents a majority of the composition, however, is known to be an inactive molecule for enhancing emulsion stability. Further, it is not cost effective to separate the inactive maltose from the active limit dextrin produced using beta-amylase. Thus, due to cost, performance and difficulty in use, there is still a need for a product which combines the properties of emulsification with stability during shelf storage, refrigeration and freeze/thaw cycles, and which may be used to replace gum Arabic.

### BRIEF DESCRIPTION OF THE FIGURES

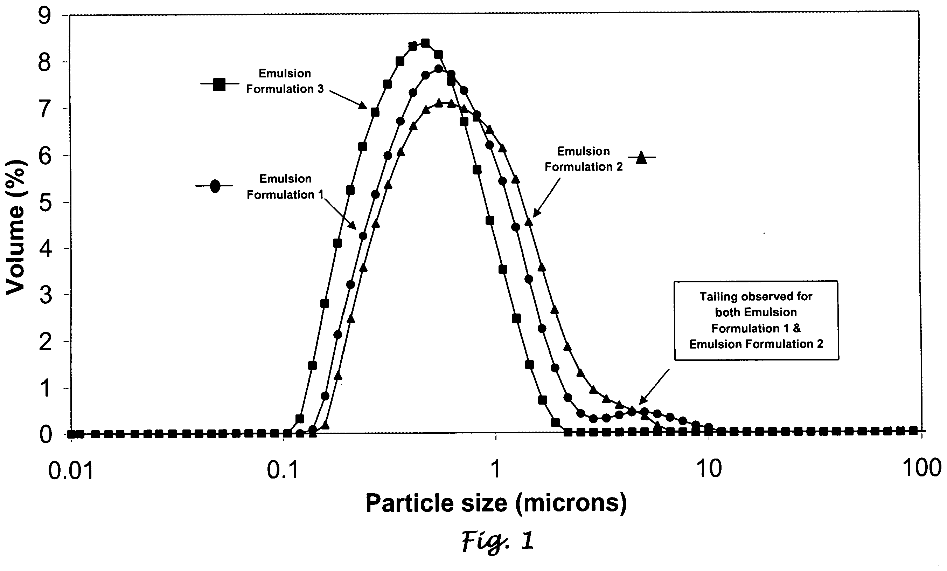

Figure 1 shows the particle size distribution of 3 Emulsion Formulations.

### SUMMARY OF THE INVENTION

The invention features a method for producing an enzymatically hydrolyzed starch. The method includes the steps of: (a) first gelatinizing a n-octenyl succinic anhydride (nOSA) starch; and then (b) hydrolyzing the gelatinized starch using a fungal alpha-amylase enzyme .

Another aspect of the invention features an enzymatically hydrolyzed starch for use as a stabilizing agent including a nOSA starch that has been hydrolyzed by an enzyme having endo-hydrolytic activity. The starch is gelatinized at a temperature between 120°C and 150°C prior to being hydrolyzed.

The resulting enzymatically hydrolyzed starch may be used to create an emulsion having superior stability for use in food, beverage, and industrial applications such as, for example, cosmetics. The present invention also pertains to compositions of beverages, food products, and industrial products that include the enzymatically hydrolyzed starch of the present invention.

Unexpectedly, in some embodiments, it was observed that the enzymatically hydrolyzed starch of the present invention remains stable to retrogradation in an aqueous mixture at starch levels of less than 50% solids by weight (preferred embodiments include those of less than 30% solids by weight, and more preferably less than 15% by weight) in a water based solvent for at least 90 days under storage conditions of less than 50°C. In other embodiments, the enzymatically hydrolyzed starch of the present invention remains stable to retrogradation under storage conditions of less than 25°C, and in yet other embodiments less than 10°C. In a particular embodiment the enzymatically hydrolyzed starch of the present invention remains stable to retrogradation in an aqueous solution at starch levels of between 25% to 35% solids by weight in a water based solvent for at least 90 days under storage conditions of less than 10°C.

In some embodiments, when used to create an emulsion, the enzymatically hydrolyzed starch surprisingly remains stable to retrogradation at starch levels of less than 50% solids by weight in an aqueous solution for at least 90 days under storage conditions of less than 50°C. In a particular embodiment, when used to create an emulsion, the enzymatically hydrolyzed starch remains stable to retrogradation in an aqueous solution at starch levels of between 10% to 15% solids by weight in a water based solvent for at least 295 days under storage conditions less than 30°C. In another particular embodiment, when used to create an emulsion, the enzymatically hydrolyzed starch remains stable to retrogradation in an aqueous solution at starch levels of between 5% to 15% solids by weight in a water based solvent for at least 180 days under storage conditions less than 10°C.

In yet another embodiment, the enzymatically hydrolyzed starch is used to create an emulsion that includes oil droplets of mono-modal and predominantly Gaussian particle size less than 5 micrometers. In this embodiment, the average particle size is maintained within 10% of its initial value for at least 90 days under storage conditions of less than 50°C.

The starch used in the invention is a n-octenyl succinic anhydride starch. The enzyme used in the invention is a fungal alpha-amylase.

In the above aspects, the starch is gelatinized by exposing the starch to temperatures between 120°C and 150°C. In some constructions the gelatinized starch is cooled to between 40°C and 60°C before it is hydrolyzed.

The foregoing and other objects and features of the disclosure will become more apparent from the following detailed description.

### DETAILED DESCRIPTION OF THE INVENTION

### I. Introduction

The present invention comprises a method to produce an enzymatically hydrolyzed starch for use as a stabilizing agent. The resulting product and applications of the resulting product may be used in food, beverage, and industrial applications. The process includes the steps of: first, gelatinization of a starch; and next, hydrolysis of the gelatinized starch using an enzyme having endo-hydrolytic activity. Applications of the resulting enzymatically hydrolyzed starch include emulsions for use in beverage, food and industrial applications. Emulsions prepared with the enzymatically hydrolyzed starch of the present invention remain surprisingly stable to retrogradation under cold storage conditions and even during freeze/thaw cycles. Accordingly, the enzymatically hydrolyzed starch of the present invention can be used to replace gum Arabic and other modified starches currently used for emulsions in such beverage, food, and industrial applications.

### II. Abbreviations and Terms

The following explanations of terms and methods are provided to better describe the present disclosure and to guide those of ordinary skill in the art in the practice of the present disclosure. As used herein, "comprising" means "including" and the singular forms "a" or "an" or "the" include plural references unless the context clearly dictates otherwise. The term "or" refers to a single element of stated alternative elements or a combination of two or more elements, unless the context clearly indicates otherwise. The term percent solids as used in a mixture or solution herein refers to percent by weight of the respective mixture or solution.

Although methods and materials similar or equivalent to those described herein can be used in the practice or testing of the present disclosure, suitable methods and materials are described below. The materials, methods, and examples are illustrative only and not intended to be limiting. Other features of the present invention are apparent from the following detailed description and the claims.

Definitions of common terms in chemistry may be found in Richard J. Lewis, Sr. (ed.), Hawley's Condensed Chemical Dictionary, published by John Wiley & Sons, Inc., 1997 (ISBN 0-471-29205-2).

The term "emulsion", as used herein, means a stable dispersion of one fluid in a second immiscible liquid, such as for example oil in water. In an oil in water emulsion, the emulsion typically includes an unweighted oil, water, and a food grade stabilizer. The unweighted oil component can be any unweighted digestible or nondigestible oil from any animal or vegetable source, including for example, terpene hydrocarbons, vegetable oils, flavor oils, nondigestible polyol polyesters or mixtures thereof. An emulsion can also include a weighted oil such that the density of the oil phase is matched with the water phase and the tendency to separate is reduced. Weighting agents can include, for example, sucrose acetate isobutyrate, brominated vegetable oil, ester gum and other ingredients. An emulsion concentrate is an emulsion produced using the above mentioned ingredients with the purpose of providing a concentrated form for later dilution into a finished beverage. For instance, a flavor oil, starch and water can be combined and made into an emulsion concentrate. Additional water, sugar, carbonation and preservative can be later mixed in to the concentrate to make a finished beverage product. Industry often seeks to use the highest oil and starch concentration possible in the emulsion concentrate to facilitate high production throughput, high strength flavor concentrates, and to minimize costs.

Retrogradation of starches refers to a crystallization like process that occurs when linear portions of the starch molecules align themselves next to each other and form interchain hydrogen bonds through hydroxyl groups. When sufficient interchain bonding occurs, the molecules associate to form molecular aggregates, which display a reduced capacity for hydration and, therefore, lower water solubility. These aggregates may precipitate, or, in more concentrated solutions, may form a gel. The tendency to retrograde is more pronounced in starches containing high levels of the linear amylose molecule. In starches containing both linear (amylose) and branched (amylopectin), and even more so with starches containing only branched molecules, the tendency to retrograde is less pronounced. Regardless, as the temperature is lowered, both amylose and amylopectin containing starches display a greater tendency to retrograde. The tendency to retrograde increases with increasing starch concentration. Once the starch fraction retrogrades, the emulsion stabilizing capacity of the starch is reduced or eliminated and the emulsion fails. It is therefore important to provide a starch for emulsion stabilization that has a low tendency to retrograde at reduced temperature, under high concentration and for long storage times.

The term "stable", as used herein, means the emulsion or water phase mixture does not show a significant change in properties with respect to time. It is an important parameter for emulsion concentrates that they retain their material properties between the time of production and the time they are diluted into a finished beverage. Furthermore, the finished beverage must be shelf-stable between the time of production and its final consumption. Oil droplet particle size, viscosity, transmittance and reflectance of light, are all properties which must remain stable in order for an emulsion to perform as expected. A stable emulsion refers to an emulsion that for instance, does not have a shift in the average oil particle size greater than 10% of its initial value, nor does it show any significant visible defect such as flocculation, sedimentation, or ringing. Stable to retrogradation means the starch does not appreciably recrystallize or precipitate out of solution during storage. Typical indicators for retrogradation include an increase or decrease in whiteness and reflectivity to light over time, an increase or decrease in viscosity, or an increase in crystallinity as measured by increasing enthalpy of melting during differential scanning calorimetry. An emulsion stabilizer is an ingredient added to a formulation to enhance the stability of the emulsion properties.

Two measurements are particularly useful in determining when an enzymatically hydrolyzed starch in an aqueous solution is no longer stable to retrogradation. The first is the visual observation of a liquid to solid transition of the enzymatically hydrolyzed starch in an aqueous solution. The second is measurement of 0% average transmittance between 10mm and 30mm using a Turbiscan™ turbimeter on a sample of the enzymatically hydrolyzed starch in an aqueous solution. These measurements are similarly useful when used to analyze when an emulsion including an enzymatically hydrolyzed starch is no longer stable to retrogradation.

An enzyme is a protein that catalyzes a biochemical reaction. An amylase is an enzyme which hydrolyzes starch. An alpha-amylase is an enzyme that catalyses the endo-hydrolysis of 1-4-alpha-glycosidic linkages in starch, glycogen, and related polysaccharides and oligosaccharides containing 3 or more 1,4-alpha-linked d-glucose units. The alpha-amylase is fungal.

The term "endo-hydrolytic activity", as used herein, refers to enzyme activity carried out with an enzyme that can cleave bonds which are internal to the molecule. These bonds include α-1,4 glucosidic linear linkages in the case of alpha amylases as well as α-1,6 glucosidic branching points in the molecule in the case of other enzymes such as pullulanases and isoamylases. As an example only, an Endoenzyme is an endoamylase capable of rapidly hydrolyzing the interior (α-1,4 glucosidic) linkages of gelatinized starch, amylose, and amylopectin solutions yielding soluble dextrins with lesser quantities of glucose and maltose. The fungal alpha-amylase enzyme CLARASE® L 40,000 (available from Genencor International) is an example of an enzyme which is capable of endo-hydrolytic action. The above description of endo-hydrolytic activity contrasts with the action of known exo-hydrolytic enzymes, such as beta-amylase, in that endo-hydrolytic enzymes do not only cleave beta-maltose units from the non-reducing end of the starch molecules as beta-amylase does. The endo-hydrolytic enzymes of the present invention are fungal alpha-amylases. The predominant enzyme activity is endo-hydrolytic, preferred are the enzymes in which the primary enzymatic activity is endo-hydrolytic, or those in which the substantially all enzymatic activity is endo-hydrolytic.

The term "gelatinize", as used herein, means the irreversible disruptions of the molecular orders within the starch granule. The process of gelatinization turns starch from a suspension of insoluble semi-crystalline granules to a swollen amorphous hydrogel or with further heat and shear into an aqueous dispersion of soluble and semi-soluble polysaccharide molecules.

The term "hydrolyze", as used herein, means to cleave a polymer under the action of acid, enzyme, heat, shear or combination thereof. The mode and rate of hydrolysis and therefore the composition of the resulting product is related to the type of enzyme used, the concentration of substrate and exposure time among other parameters. In general, starch hydrolysis is accompanied by a reduction in molecular weight and viscosity of the polymer.

n-octenyl succinic anhydride ("nOSA") is a reagent that can be used to modify a starch. Treatment of starch with nOSA results in a modified starch that has both hydrophilic and hydrophobic moieties. The resulting nOSA starch can aid in emulsification. An exemplary nOSA starch fragment is shown below: 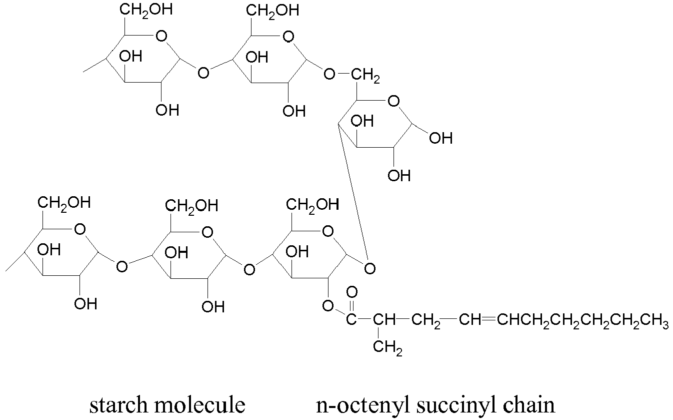 A starch is a carbohydrate polymer. Starches consist essentially of amylose and/or amylopectin and in the native form are typically in the form of semi-crystalline granules. Amylopectin is the major component (about 70%-80%) of most starches. It is a branched polymer of several thousand to several million glucose units. Amylose is the minor component (about 20%-30%) of most starches. However, there are high amylose starches with 40%-90% amylose. Amylose is composed of more linear glucose polymers with some long chain branching and consists of several hundred to several hundred thousand glucose units. Waxy starches are composed of mainly amylopectin molecules.

Sources of starch include but are not limited to fruits, seeds, and rhizomes or tubers of plants. Typical sources of starch include but are not limited to rice, wheat, corn, potatoes, tapioca, arrowroot, buckwheat, banana, barley, cassava, kudzu, oca, sago, sorghum, sweet potatoes, taro and yams. Edible beans, such as favas, lentils and peas, are also rich in starch.

Some starches are classified as waxy starches. A waxy starch consists essentially of amylopectin and lacks an appreciable amount of amylose. Typical waxy starches include waxy maize starch, waxy rice starch, waxy potato starch, and waxy wheat starch.

Some starches are classified as high amylose starches. These would include any starch having greater than the typical 15-30% apparent amylose.

The term "instant starch", as used herein, means a starch that swells or forms a colloid or dispersion of molecules or swollen hydrated granules and often develops increased viscosity in solution without heating. Instant starches are used, for example, in instant puddings. An instant starch can consist of a swollen hydrogel in solution or a soluble dispersion of molecules depending upon the modification and processing techniques employed.

The term "modified starch", as used herein, means a starch which has a structure that has been altered from its native state, resulting in modification of one or more of its chemical or physical properties. Starches may be modified, for example, by enzymes, by heat treatment, oxidation, or reaction with various chemicals including, but not limited to, propylene oxide, and acetic anhydride, or complexed with compounds including proteins. Starches may be modified to increase stability against heat, acids, or freezing. Starches may also be modified to improve texture, increase or decrease viscosity, increase or decrease gelatinization times, and/or increase or decrease solubility, among others. Modified starches may be partially or completely degraded into shorter chains or glucose molecules. Amylopectin may be debranched. In one example, modified starches are cross-linked to improve stability. Starches that are modified by substitution have a different chemical composition. A nOSA starch is a modified starch that has been partially substituted, e.g., from about 0.1% to about 3%, with n-octenyl succinic anhydride. Other modified starches include, but are not limited to, crosslinked starches, acetylated and organically esterified starches, hydroxyethylated and hydroxypropylated starches, phosphorylated and inorganically esterified starches, cationic, anionic, nonionic, and zwitterionic starches, and succinate and substituted succinate derivatives of starch. Such modifications are known in the art, for example in Modified Starches: Properties and Uses, Ed. Wurzburg, CRC Press, Inc., Florida (1986).

In contrast to a "modified starch", the term "unmodified starch", as used herein, refers to a starch whose structure has not been altered from its native state.

The term "substitution", as used herein, means the act, process, or result of replacing one thing with another. Substitution may refer, for example, to the substitution of starch for a hydrocolloid in a beverage product, such as a soda pop. Substitution may alternatively refer to the addition of one or more functional groups onto a molecule or substrate as a result of a chemical or physical or mechanical reaction. For example, n-octenyl succinic anhydride may be used in a substitution reaction with starch to produce a nOSA-modified starch.

The term "soluble", as used herein, means to be suspended, solvated, and/or molecularly dispersed in a solvent such that the concentration of the solute in the solvent is relatively homogenous throughout the volume. Soluble starch can be mixed into a solvent and becomes suspended without the addition of heat, shear or chemicals. A starch which remains soluble in an aqueous solution does not have a tendency to retrograde as an insoluble crystallite or precipitate. Similarly, a starch which retains solubility does not transition from a liquid solution to a gelled solid.

A "pregelatinized starch", as used herein, refers to a starch which has been partially or completely gelatinized and recovered as a dry particle which can be solubilized without additional heating. Typical pregelatinized starch is produced by steam cooking in the nozzle of a spray dryer, by contact heating on the surface of a drum dryer, by direct steam injection in a vessel or jet cooker, or by chemical gelatinization, such as with sodium hydroxide.

### III. Methods of Preparing Enzymatically Hydrolyzed Starch

In one aspect of the present invention, the enzymatically hydrolyzed starch is prepared by forming a slurry of starch in an aqueous solution. The slurry includes the starch and water in any proportion necessary to achieve the desired enzyme-substrate concentration when the enzyme treatment is ultimately carried out. In general, the preferred enzyme hydrolysis reaction is carried out at the highest solids content that is feasible to facilitate subsequent drying of the starch composition while maintaining optimum reaction rates. For example, a precooked starch dispersion at a solids content ranging up to 40% is suitable for production of an emulsifier for beverage applications using a fungal alpha-amylase. However, a higher solids content may be used. But at a higher solids content agitation is difficult or ineffective and the starch dispersion is more difficult to handle.

The slurry of starch in an aqueous solution is cooked to gelatinize the starch. The starch can be gelatinized according to a number of methods including, for example, solubilization through thermal gelatinization, chemical gelatinization, mechanical treatment or imposition of other energy forms. In a particular embodiment, the slurry is cooked to substantially completely gelatinize the starch. The gelatinization process disrupts the starch molecules from the granular structure, which permits the enzyme to more easily and uniformly hydrolyze the starch molecules. The slurry is cooked to temperatures of between 120°C and 150°C. In one embodiment of the present invention, the thermal treatment is such that temperature and shear cause a substantially complete molecular dispersion of the starch, substantially free from residual granular structure, and residual crystalline junction zones. In this aspect, the high temperature thermal treatment minimizes or removes the residual structures, which could later act as nucleation sites for crystallization and other starch destabilizing processes.

Any cooking method capable of cooking the slurry to and holding the slurry at the appropriate temperatures can be used. For example, an appropriate holding time, or residence time, is an amount of time sufficient to ensure complete gelatinization of the starch. The type of starch can also have an impact of the appropriate cooking method. For example, an instant starch will not require significant additional cooking or as high of a temperature as a modified granular starch such as a nOSA, waxy uncooked starch. In another embodiment, a chemical treatment can be used either in addition to or in place of cooking to gelatinize the starch derivative. Suitable chemicals for the present invention may include, for example, sodium hydroxide or potassium hydroxide. U.S. Patent No. 4,985,082 to Whistler, Roy L., also reports methods of degrading granular, non-cooked starched.

Hydrolysis of the starch using an enzyme having endo-hydrolytic activity is carried out after gelatinization of the starch. A number of factors, including enzyme concentration, substrate concentration, pH, temperature, and the presence or absence of inhibitors can affect activity of the enzyme. The parameters for optimum enzyme activity will vary depending upon the enzyme used and can be determined by one of skill in the art. For instance, following gelatinization of the starch, the pH, temperature or both the pH and temperature of the gelatinized starch should be adjusted for optimum enzyme activity. In another instance, the temperature of the gelatinized starch should be adjusted for the particular enzyme to be used during hydrolysis. Thus, in one aspect of the present invention, the gelatinized starch is cooled to a temperature of between 40°C and 60°C prior to hydrolysis using an enzyme. In another aspect of the present invention, the pH of the gelatinized starch is adjusted to between 4.0 and 6.0. In still another aspect of the present invention, the gelatinized starch is both cooled to between 40°C and 60°C and the pH of the gelatinized starch derivative is adjusted to between 4.0 and 6.0. The reaction may proceed in the presence of buffers to ensure that the pH will be at the optimum level throughout the hydrolysis. Buffers such as acetates, citrates, or the salts of other weak acids are acceptable. In some instances, introducing materials such as calcium or sodium can increase the half-life of the enzyme, thus further optimizing enzyme activity. As with other parameters of the enzyme reaction, optimum temperature ranges will vary with changes in other parameters such as substrate concentration, pH and other factors affecting enzyme activity, and can be determined by the practitioner.

Although the process of this invention makes use of an enzyme in solution, processes utilizing an enzyme immobilized on a solid support are intended to fall within the scope of this invention.

The enzyme reaction is permitted to continue until the desired level of hydrolysis is reached in the starch. In one embodiment of the present invention, starch should be held at a temperature of from 35°C to 70°C for approximately 20 minutes during the enzymatic digestion. If shorter reaction times are desired, a temperature range of from 50°C to 55°C may be used. Alternately, a higher enzyme concentration may be used. The progress of enzyme reaction may be measured by various methods. If specific parameters have been established for achieving a particular starch composition, then the reaction may be allowed to proceed to a predetermined relative end point in time. The end point also may be monitored and defined by measuring the concentration of reducing sugars. Other techniques such as monitoring the change in viscosity, spectral changes, or the change in molecular weight may be used to define the reaction end point.

The hydrolysis will be carried out for periods ranging from a few minutes to 24 hours or more depending on the temperature, enzyme and substrate concentrations, and other variables. The enzyme action is then terminated by means of heat, chemical additions, or other methods known in the art for deactivating an enzyme or separating an enzyme from its substrate. In one aspect of the present invention, the fungal alpha-amylase promotes a faster reaction rate compared to beta-amylase and also leads to higher production rates. For instance, while beta-amylase reactions may require 6-8 hours of reaction time, fungal alpha-amylase produced reaction times of 20 minutes or less to reach the desired endpoint. Several benefits can result from the reduced reaction time, including easier adaptation to a continuous process, minimizing tank sizes and minimization of degradation to the substituted groups on the starch molecule. Adaptation to a continuous and short time process, for example less than 1 hour, provides additional benefits, including the ability to preserve active substituent groups on the starch derivative such as, for example, succinate esters, at lower processing temperatures than what would be necessary using a batch process or a longer residence time process, such as for example more than 1 hour. Long residence times and batch processes can cause hydrolysis of functional groups and make the starch inactive for the purpose of emulsifying.

The resulting enzymatically hydrolyzed starch for use as a stabilizing agent may be spray-dried, drum-dried or otherwise recovered in a form suitable for the intended application. If the end-use application requires purification of the starch composition, sugars and other reaction impurities and by-products may be removed by dialysis, filtration, centrifugation or any other method known in the art for isolating and concentrating starch compositions. The enzymatically hydrolyzed starch of the present invention may also be supplied in liquid form, including a concentrated liquid form, without further drying or recovery.

### IV. Applications of the Enzymatically Hydrolyzed Starch

### A. Emulsions

The enzymatically hydrolyzed starch of the present invention has application in a number of emulsions, including those previously described.

The emulsions can be prepared using methods known to those skilled in the art, except that the enzymatically hydrolyzed starch of the present invention is added.

### B. Beverages. Food Products, and Industrial Products

The enzymatically hydrolyzed starch of the present invention may be used in a variety of applications, including any product where gum Arabic has been used as an emulsifier, stabilizer, or the like, and in any product where high molecular weight, water soluble emulsifiers, including certain modified starches, have been used to form or stabilize emulsions.

In one aspect, the enzymatically hydrolyzed starch of the present invention may be used in beverages that are flavored with oils such as orange or lemon oils, confectionery items, ice cream, other beverages and other food products which require a shelf stable emulsifier. It may further be used in water-and-alcohol based beverages.

The enzymatically hydrolyzed starch of the present invention can also be used in preparing encapsulated spray-dried or extruded flavor oils that are reconstitutable with water to provide flavor emulsions, seasonings as well as in inks, textiles and other non-food end uses.

As another example of its application, the enzymatically hydrolyzed starch of the present invention may be used in the production of shelf stable bakery products, where the emulsifying capacity and anti-staling functionality of the enzymatically hydrolyzed starch is exploited. The enzymatically hydrolyzed starch also exhibits anti-staling activity in bakery products such as bread. For instance, a hydrophobic group in one embodiment of the invention can interact with the amylose and amylopectin present in bread flour to prevent unwanted changes in texture, crumb, edibility and salability.

The enzymatically hydrolyzed starch of the present invention can further be used in the emulsification of meat products and additives to meat products. In one aspect, the enzymatically hydrolyzed starch reduces purge when used in combination with viscosifiers and water binding additives.

The enzymatically hydrolyzed starch of the present invention can also be used as a stabilizer and texturizer in dairy and smoothie products. In at least one aspect, the enzymatically hydrolyzed starch provides suitable shelf stable structure and reduces syneresis or other undesirable textural changes to the product.

The enzymatically hydrolyzed starch of the present invention can also find application in the production of carotene or Vitamin E emulsification and encapsulation as coloring or nutritive ingredients. Benefits of using the enzymatically hydrolyzed starch for this purpose include long-term solution stability of the emulsion.

In yet another example, the enzymatically hydrolyzed starch can be used in the emulsification and/or encapsulation of probiotics, prebiotics, and dietary supplements. In a particular example, the enzymatically hydrolyzed starch of the present invention can be used in an Omega 3 fatty acid emulsion.

It is to be understood that the invention herein includes any emulsified composition wherein the emulsifying agent is starch that has been enzymatically hydrolyzed to improve shelf stability of an emulsion. Thus, it is meant to include emulsions comprising a blend of the enzymatically hydrolyzed starch and gums or other emulsifying agents. The invention also includes any non-emulsified composition wherein the starch is utilized as a texturizing agent or to preserve the texture of the composition.

### EXAMPLES

The following examples will more fully illustrate the embodiments of the present invention. It is understood that these examples are not intended to limit the scope of the present invention in any way. In these examples, all parts and percentages are given by dry weight basis and all temperatures are in degrees Celsius unless otherwise noted. Shelf stability is measured at low temperature to accelerate retrogradation and shorten the testing period.

Examples 1 and 2 provide process steps to prepare an enzymatically hydrolyzed starch according to the present invention.

### Example 1: Preparation of an Enzymatically Hydrolyzed Starch (Continuous Process)

### Step 1: 15% Starch Slurry Preparation

5,500 lbs of EmTex 06369, a nOSA waxy starch available from Cargill, Incorporated, was obtained. The starch was slurried in a tank with 3178 gallons water to 15% solids. The starch was added incrementally up to 5,500 lbs of starch, and during addition of the starch, cold filtered water was added up to 3197 gallons to suspend the starch.

Next, 14 lbs of 32% calcium chloride and 5.95 lbs of 35% bisodium sulfite were added to the mixture. The mixture was allowed to stir overnight. 4.05 lbs of 35% bisodium sulfite was added to the mixture the following day. Finally, the pH of the solution was checked and adjusted to 5.57 using sodium hydroxide.

### Step 2: Gelatinization (Starch Cooking)

The starch was cooked at about 143°C using a Hydrothermal™ jet cooker. The residence time in the Hydrothermal™ jet cooker chamber was approximately 80 seconds. The gelatinized starch was pumped through a flash cooler and the temperature was adjusted to 57°C.

### Step 3: Hydrolysis (Enzyme Liquefaction)

CLARASE® L 40,000, a fungal alpha-amylase enzyme, was obtained from Genencor, International. The enzyme was diluted to 1:100 with DI water.

The diluted fungal alpha-amylase enzyme was continuously added to the gelatinized starch and adjusted in rate to hydrolyze the gelatinized starch to a viscosity of 14-18 cPs. The gelatinized starch was pumped through a series of continuously stirred reactor vessels with a residence time of around 20 minutes. The total time to hydrolyze 5500 lbs of starch was about 6 hours.

### Step 4: Enzyme Deactivation and Neutralization

The enzyme hydrolyzed starch was pumped into a deactivation tank where sulfuric acid was added to adjust the pH to 3.0 to deactivate the enzyme. After enzyme deactivation, 5 gallons of 10% sodium hydroxide was added, raising the pH to 3.7.

### Step 5: Spraydrying

The enzymatically hydrolyzed starch was adjusted to pH 4.5 with sodium hydroxide. Finally, the enzymatically hydrolyzed starch was spray dried to recover a dry powder.

### Example 2: Preparation of an Enzymatically Hydrolyzed Starch (Batch Process)

### Step 1: 15% Starch Slurry Preparation

Starch (50 pounds of EmTex 06369, a nOSA modified granular starch obtained from Cargill, Incorporated) was slurried in water to 15% solids. The pH was checked and adjusted to pH 6.0 with dilute sulfuric acid and dilute sodium hydroxide.

### Step 2: Gelatinization (Starch Cooking)

The starch was cooked using a Schlick™ jet cooker with a residence time in the cooker chamber of about 10 seconds at about 145°C. The gelatinized starch was pumped through a flash cooler to adjust the temperature to 53°C in preparation for enzyme addition.

### Step 3: Hydrolysis Enzyme Liquefaction)

The jet cooked starch was collected in a stirred vessel and agitated for about 70 minutes. A fungal alpha-amylase enzyme (CLARASE® L 40,000 obtained from Genencor International) was then added to hydrolyze the gelatinized starch for a total incubation time of the 15 minutes.

### Step 4: Enzyme Deactivation and Neutralization

The enzyme hydrolyzed starch was pumped into a deactivation tank where sulfuric acid was added to adjust the pH to 3.0 to deactivate the enzyme.

### Step 5: Spraydrying

The enzymatically hydrolyzed starch was adjusted to pH 4.0 with dilute sodium hydroxide and spray dried to recover a dry powder.

### Example 3: Average Emulsion Particle Size

This example illustrates a comparison of average emulsion particle size between an emulsion made using the enzymatically hydrolyzed starch produced in Example 2 and an emulsion made using gum Arabic.

A first emulsion was prepared in the following manner: The enzymatically hydrolyzed starch produced in Example 2 was mixed into water at 12% solids. Next, 18% cold pressed orange oil was added to the enzymatically hydrolyzed starch and water and blended at high speed in a blender to make a coarse emulsion. This coarse emulsion was then homogenized at 3500 psi to create a fine emulsion.

A second emulsion was made according to the procedure described above, except that instead of using the enzymatically hydrolyzed starch produced in Example 2, an emulsion grade gum Arabic was used.

A Horiba™ LA-300 Laser Scattering particle size distribution analyzer was used to determine the average emulsion particle size. 2-3 drops of the emulsion were added into water while stirring until the transmittance was between 80-90%.

A comparison of average emulsion particle size over a period of time for each of the emulsions is presented in Table 1 below.

**Table 1**

| Storage Time at Room Temperature (Days) | Average Emulsion Particle Size (µm) | |
|---|---|---|
| | Emulsion Prepared Using the Enzymatically Hydrolyzed Starch Produced in Example 2 | Emulsion Prepared Using Gum Arabic |
| 1 | 0.47 | 0.82 |
| 10 | 0.48 | 0.94 |
| 35 | 0.47 | 1.23 |
| 55 | 0.48 | 1.85 |

As seen in Table 1, the average emulsion particle size of the enzymatically hydrolyzed starch produced in Example 2 remained constant compared to the increase in the average particle size over time of the emulsion prepared using gum Arabic. An increase in particle size indicates emulsion instability (i.e. failure of the emulsion). Thus, the present invention creates a more shelf stable emulsion than one in which gum Arabic was used.

In addition, the average emulsion particle size of the emulsion prepared using the enzymatically hydrolyzed starch solution produced in Example 2 was measured after 295 days. This average emulsion particle size was measured at 0.48 µm. This data indicates a considerable shelf stable emulsion is obtained by using the enzymatically hydrolyzed starch produced in Example 2.

### Example 4: Stability to Retrogradation in Aqueous Solutions

Three enzymatically hydrolyzed starch samples were prepared from commercially available nOSA-starch using various enzymes. The pH and temperature conditions were adjusted to be suitable for the particular enzyme used.

The 3 enzymes used were BAN™ 480L (obtained from Novozymes) to yield Example 4A, SPEZYME PRIME™ to yield Example 4B, and CLARASE® L 40,000 (latter 2 enzymes were obtained from Genencor International, Inc.) to yield Example 4C. BAN™ 480L and SPEZYME PRIME™ are bacterial alpha-amylase enzymes while CLARASE® L 40,000 is a fungal alpha-amylase.

The pH and temperature conditions used during enzyme hydrolysis for each sample were the following. BAN™ 480L: pH 6.0 and 80°C; SPEZYME PRIME™: pH 6.0 and 80°C; CLARASE® L 40,000: pH 5.3 and 53°C. For the samples made with BAN™ 480L and SPEZYME PRIME™, the enzyme was added to the starch slurry prior to gelatinization and hydrolysis. The starches were cooked at 80°C through a jet cooker and hydrolyzed for 20 minutes. Sulfuric acid was added to deactivate the enzyme at pH 3.0. The pH was adjusted to between 3.5 and 4.5 with sodium hydroxide prior to spray drying. The same process was not possible using the CLARASE® L 40,000 because this enzyme becomes deactivated at temperatures greater than about 70°C. Starch cannot be fully gelatinized below 70°C. Therefore, the process according to Example 2 was used to make the sample hydrolyzed by CLARASE® L 40,000.

3 solutions were made, one solution for each enzymatically hydrolyzed starch sample. The solutions were made at 30% solids in water and tested over time for retrogradation using visual observation of liquid to solid transition. A Turbiscan™ turbidimeter was also used to measure the change in average light transmittance in samples from 10-30 mm height, which is related to the degree of opacity due to retrogradation. The samples were cycled from a temperature of about 4°C to 25°C daily. The samples were considered unstable when either a liquid to solid transition was visually observed by tilting the sample vial to the horizontal position, or when the Turbiscan™ turbidimeter measured 0% average transmittance between 10mm and 30mm in the sample vial. These two measurements yielded the same outcome of Stability to Retrogradation (Days) for each sample. The data is presented in Table 2.

**Table 2**

| Enzyme used to Prepare Enzymatically Hydrolyzed Starch Sample Solution: | Stability to Retrogradation (Days) |
|---|---|
| 4A) BAN™ 480L | < 20 |
| 4B) SPEZYME PRIME™ | < 10 |
| 4C) CLARASE® L 40,000 | > 50 |

As seen in Table 2, the solution made using a starch produced by gelatinizing at high temperature, cooling and then enzyme hydrolysis with the fungal alpha-amylase enzyme, remained stable to retrogradation longer than 50 days. In contrast, the solutions made using a starch produced by gelatinizing with bacterial enzymes present at lower temperature around 80°C remained stable for less than 20 days.

### Example 5: Stability to Retrogradation in Representative Beverage Formulations

Two beverages, Beverage 1 and Beverage 2, were prepared according to the recipe in Table 3, but using different enzymatically hydrolyzed starch samples.

**Table 3**

| Ingredient | Total % Basis | Total Weight (g) |
|---|---|---|
| Enzymatically Hydrolyzed Starch Sample | 30% | 240 |
| Citric Acid (30%) | 0.25% | 2 |
| Sodium Benzoate (50%) | 0.10% | 0.8 |
| Vitamin C | 0.15% | 1.2 |
| Water | 69.50% | 556 |
| Total | 100% | 800 |

In Beverage 1, the enzymatically hydrolyzed starch sample used was made with BAN™ 480L enzyme as in Example 4A. In Beverage 2, the enzymatically hydrolyzed starch sample used was the enzymatically hydrolyzed starch prepared according to the process described in Example 2.

To prepare each beverage, the water was first brought to 65°C in a double jacketed beaker connected with a water bath. The remaining ingredients were then added to the water. Each mixture was allowed to hydrate for 2 minutes. During this time, the mixtures were stirred at a speed of 400 rpm. The two beverage mixtures were poured into separate cups and refrigerated. Viscosity measurements of the refrigerated beverage mixtures were taken over time using a Haake Rheostress 1. Table 4 shows the viscosity of each beverage over time at a shear rate of approximately 1 s⁻¹.

**Table 4**

| | Viscosity (cP) | | | |
|---|---|---|---|---|
| | Day 1 | Day 3 | Day 6 | Day 9 |
| Beverage 1 (measured at a shear rate of 1.062 s⁻¹) | 1,160 | 1,490 | 3,970 | 101,000 |
| Beverage 2 (Measured at a shear rate of 1.067 s⁻¹) | 964 | 909 | 931 | 880 |

Viscosity over time is an indicator of the degree of stability of a starch. As seen in Table 4, the viscosity of Beverage 2, the beverage using the enzymatically hydrolyzed starch produced in Example 2, remained relatively constant in comparison to the Beverage 1. It should be noted that the enzymatically hydrolyzed starch produced in Example 2 was made using a starch produced by gelatinizing at high temperature, cooling and then enzyme hydrolysis with CLARASE® L 40,000, a fungal alpha-amyalse enzyme with endo-hydrolytic activity.

In contrast, the enzymatically hydrolyzed starch used in Beverage 1 was a starch gelatinized with a bacterial enzyme present at a lower temperature of around 80°C. Beverage 1 suffered significant retrogradation over time as can be seen from its increasing viscosity measurements in Table 4. In fact, after 9 days, Beverage 1 became a white jelly product, almost sliceable, and very difficult to measure.

### Example 6: Emulsion Concentrate Stability

Two beverage emulsion concentrates, Beverage 1 and Beverage 2, were prepared according to the recipe in Table 5, but using different enzymatically hydrolyzed starch samples.

**Table 5**

| Ingredient | Concentration (%) | Total Weight (g) |
|---|---|---|
| Enzymatically Hydrolyzed Starch Sample | 10.00 | 50.00 |
| Orange Peel Oil | 10.00 | 50.00 |
| Sodium Benzoate (30%) | 0.67 | 3.33 |
| Citric Acid (50%) | 0.60 | 3.00 |
| Water | 78.73 | 393.67 |
| Total | 100 | 500 |

In Beverage 1, the enzymatically hydrolyzed starch sample used was made with enzyme BAN™ 480L as in example 4. In Beverage 2, the enzymatically hydrolyzed starch sample used was the enzymatically hydrolyzed starch prepared according to the process described in Example 2.

Each of the 2 Beverages was made as follows. First, the enzymatically hydrolyzed starch sample was mixed into 350 ml water containing sodium benzoate at 60°C. The mixture was kept at 60°C for 2 hours and mixed at regular intervals. The mixture was cooled down to room temperature and citric acid was added to adjust the pH to 3. Water was then added to bring the mixture to 450g. Next, the orange peel oil was added while mixing using an Ultra-Turrax (speed 2). Mixing was continued for another 3 minutes. Immediately after mixing, the emulsion was homogenized in 2 stages (2500/500 psi or 175/35 Bar) and passed through the homogenizer twice. The final pH was checked and adjusted to pH 3.0-3.5, if necessary.

The Beverages were stored at about 6°C and pH of about 3.0 to about 3.5. Emulsion stability of the 2 Beverages was measured using a Turbiscan™ Instrument to determine the average backscattering of the solution between 10mm and 30mm in the vial of sample. The higher the reduction in backscattering over time, the less stable the emulsions during storage. A decrease in backscattering in the middle of the sample indicates the emulsion is destabilizing with oil droplets migrating to the top or bottom of the vial. The measurements indicated that Beverage 2 had a stable emulsion at 180 days with an average backscattering decrease of less than 20% while Beverage 1 maintained a stable emulsion for less than 50 days with an average backscattering decrease of greater than 20%.

The emulsion stability of Beverage 2 was significantly better than emulsion stability of Beverage 1. Beverage 2 was also the Beverage containing the a starch produced by gelatinizing at high temperature, cooling and then enzyme hydrolysis with CLARASE® L 40,000, a fungal alpha-amyalse enzyme with endo-hydrolytic activity. The enzymatically hydrolyzed starch in Beverage 1 was made with a starch gelatinized with a bacterial enzyme present at a lower temperature of around 80°C.

### Example 7: Stability with different enzymes and with gelatinization before enzyme liquefaction

The purpose of this example was to demonstrate the necessity of combining gelatinization at high temperature followed by fungal alpha amylase enzyme hydrolysis. 2 Beverages were prepared according to the recipe in Table 3, but using different enzymatically hydrolyzed starch samples. Each enzymatically hydrolyzed starch sample was prepared according to the process of Example 2 except using different starch and enzyme combinations. The enzymatically hydrolyzed starch sample of Beverage 1 was made using the starch Emtex 06369 and the enzyme BAN™ 480L. The enzymatically hydrolyzed starch sample of Beverage 2 was made using the starch Emtex 06369 and the enzyme CLARASE® L 40,000. The 2 Beverages were then stored at about 6°C.

The emulsion stability of the 2 Beverages was thereafter studied at pH 3.5 (to represent a soda pop beverage). Stability was determined by measurement of the average transmittance between 10-30 mm using the Turbiscan™ Turbidimeter. When the transmittance reduced to zero, the sample was considered no longer stable. The resulting data is presented in Table 6.

**Table 6**

| Sample | Stability at pH 3.5 (Days) |
|---|---|
| Beverage 1 (Gelatinization at 145°C followed by BAN™ 480L) | 45 days |
| Beverage 2 (Gelatinization at 145°C followed by CLARASE® L 40,000) | > 90 days |

As shown in Table 6, the beverage containing a starch produced with BAN™ 480L did not remain stable for as long as the beverage containing a starch produced with CLARASE® L 40,000, even though both products were gelatinized first without enzyme, cooled and then the enzyme was added for hydrolysis. This data indicates that the stability of the enzymatically hydrolyzed starch is related to the enzyme type rather than solely the process steps and temperatures of liquefaction.

### Example 8: Particle Size Distribution and Average Particle Size

Three Emulsion Formulations were prepared according to the recipe in Table 7, but using different Emulsion Stabilizers.

**Table 7**

| Ingredient | | Total Weight (g) | Concentration (%) |
|---|---|---|---|
| Emulsion Stabilizer | | 150.00 | 20.00 |
| Oil Phase (density = 0.9757g/ml) | | 127.50 | 17.00 |
| Orange Peel Oil | 41% | | |
| Ester Gum | 59% | | |
| Sodium Benzoate (30%) | | 5.00 | 0.67 |
| Citric Acid (50%) | | 4.50 | 0.60 |
| Water | | 463.01 | 61.73 |
| Total (pH = 3.0-3.5) | | 750.00 | 100.00 |

The Emulsion Stabilizer used in Emulsion Formulation 1 was made with BAN™ 480L as in Example 4. The Emulsion Stabilizer used in Emulsion Formulation 2 was gum Arabic (spray dried gum acacia 393A obtained from Farbest Brands). The Emulsion Stabilizer used in Emulsion Formula 3 was an enzymatically hydrolyzed starch according to the process described in Example 2.

The Emulsion Formulations were prepared by mixing the Emulsion Stabilizer Starch into water containing sodium benzoate at 60°C. The solution was kept at 60°C for 2 hours and mixed at regular intervals. The solution was then cooled to room temperature. Next, the citric acid was added to adjust the pH to about 3. The remaining water was then added. The oil phase was next added while mixing using an Ultra-Turrax at speed 2. Mixing was then continued for another 3 minutes. The sample was left at room temperature for a sufficient amount of time to allow the foam to collapse. The emulsion was thereafter homogenized in two stages (first at 2500 psi (or 175 bar) and then at 500 psi (or 35 bar)) and passed through the homogenizer twice. The final pH was checked and adjusted to pH 3.0-3.5 as necessary.

The particle size distribution for each Emulsion Formulation is provided in Figure 1. A Mastersizer 2000 laser light scattering particle size analyzer was utilized to determine the oil droplet size in the emulsion concentrates. The analysis was done by placing 2-3 drops into a stirred water solution until the instrument transmittance was around 80%. Tailing was observed for both Emulsion Formulation 1 and Emulsion Formulation 2. Tailing is an indicator of large particles, which results in emulsion instability (i.e. failure of the emulsion). The Emulsion Formulation which did not exhibit Tailing was Emulsion Formulation 3. Emulsion Formulation 3 used an Emulsion Stabilizer which was made using the process of example 2 including gelatinization followed by cooling and enzyme liquefaction with CLARASE® L 40,000 fungal alpha-amylase.

Table 8 shows the Average Particle Size for the three Emulsion Formulations.

**Table 8**

| Emulsion Formulation | Average Diameters (µm) | | | | |
|---|---|---|---|---|---|
| | D10 | D50 | D90 | Volume Average Diameter | Surface Area Average Diameter |
| 1 | 0.26 | 0.61 | 1.50 | 0.51 | 0.84 |
| 2 | 0.30 | 0.71 | 1.83 | 0.59 | 0.95 |
| 3 | 0.22 | 0.47 | 1.04 | 0.40 | 0.56 |

D10, D50 and D90 describe the diameter below which a % of the particles lie. This can be further explained as follows: Dx with x=10, 50, and 90. The value shown is the diameter (in µm) below which x% of the volume of the particles lies. For example, in Emulsion Formulation 3, 90% of the particles are below the particle size 1.04 µm whereas in Emulsion Formulation 2, 90% of the particles are below the particle size 1.83 µm and in Emulsion Formulation 1, 90% of the particles are below the particle size 1.50 µm. This data indicates that there is a greater presence of larger particles in Emulsion Formulations 1 and 2. This greater percentage of larger particles is an indicator of emulsion instability. The data for D10, D50, Volume Average Diameter and Surface Area Average Diameter may be similarly interpreted.

Table 9 shows the Distribution Width and the Specific Surface Area for the three Emulsion Formulations.

**Table 9**

| Emulsion Formulation | Distribution Width (µm) | Specific Surface Area (m²/cc) |
|---|---|---|
| 1 | 2.04 | 11.98 |
| 2 | 2.15 | 10.20 |
| 3 | 1.74 | 14.90 |

The Distribution Width is a value obtained with the following calculation: ((D90 - D10)/D50). The Specific Surface Area is inversely related to the Surface Area Diameter. A higher Specific Surface Area represents a better emulsion. Here, the highest specific surface area is exhibited by Emulsion Formulation 3.

## Claims

1. A method for producing an enzymatically hydrolyzed starch comprising the steps of:
(a) first, gelatinizing a n-octenyl succenic anhydride starch by exposing the starch to temperatures between about 120°C and 150°C, and
(b) then, enzymatically hydrolyzing the gelatinized starch using a fungal alpha-amylase as the enzyme.

2. The method of claim 1, further comprising the step of cooling the gelatinized starch to between about 40°C and about 60°C before it is hydrolyzed.

3. An enzymatically hydrolyzed starch for use as a stabilizing agent comprising a n-octenyl succenic anhydride starch that has been gelatinized at a temperature between about 120°C and 150°C and hydrolyzed by an enzyme having endo-hydrolytic activity, wherein the enzyme is a fungal alpha-amylase.

4. An emulsion comprising the enzymatically hydrolyzed starch of claim 3.

5. The emulsion of claim 4, wherein the emulsion comprises oil droplets of mono-modal and predominantly Gaussian particles size less than about 5 micrometers and wherein the average particle size is maintained within 10% of its initial value for at least 90 days when stored at temperatures below 50°C.

6. A food, beverage or industrial product comprising the enzymatically hydrolyzed starch of claim 3 or the emulsion of claims 4 or 5.

## Patentansprüche

1. Verfahren zum Herstellen einer enzymatisch hydrolysierten Stärke, umfassend die folgenden Schritte:
(a) zuerst Gelatinieren einer n-Octenylsuccensäureanhydridstärke durch Aussetzen der Stärke Temperaturen zwischen etwa 120 °C und 150 °C, und
(b) dann enzymatisches Hydrolysieren der gelatinisierten Stärke unter Verwendung einer fungalen alpha-Amylase als Enzym.

2. Verfahren nach Anspruch 1, weiter umfassend den Schritt des Abkühlens der gelatinisierten Stärke auf zwischen etwa 40 °C und etwa 60 °C, bevor sie hydrolysiert wird.

3. Enzymatisch hydrolysierte Stärke zur Verwendung als Stabilisierungsmittel, umfassend eine n-Octenylsuccensäureanhydridstärke, die bei einer Temperatur zwischen etwa 120 °C und 150 °C gelatinisiert und durch ein Enzym mit endo-hydrolytischer Aktivität hydrolysiert wird, wobei das Enzym eine fungale alpha-Amylase ist.

4. Emulsion, umfassend die enzymatisch hydrolysierte Stärke nach Anspruch 3.

5. Emulsion nach Anspruch 4, wobei die Emulsion Öltröpfchen aus monomodalen und vorwiegend Gaußschen Teilchen mit einer Größe von weniger als etwa 5 Mikrometer umfasst und wobei die durchschnittliche Teilchengröße bei Lagerung bei Temperaturen unter 50 °C für mindestens 90 Tage innerhalb von 10 % ihres Anfangswerts gehalten wird.

6. Nahrungsmittel-, Getränke- oder Industrieprodukt, umfassend die enzymatisch hydrolysierte Stärke nach Anspruch 3 oder die Emulsion nach Anspruch 4 oder 5.

## Revendications

1. Procédé de production d'un amidon hydrolysé par voie enzymatique comprenant les étapes consistant à :
(a) premièrement, gélatiniser un amidon d'anhydride n-octényl succénique en exposant l'amidon à des températures comprises entre environ 120 °C et 150 °C, et
(b) ensuite, hydrolyser par voie enzymatique l'amidon gélatinisé en utilisant une alpha-amylase fongique comme enzyme.

2. Procédé selon la revendication 1, comprenant en outre l'étape consistant à refroidir l'amidon gélatinisé pour qu'il soit entre environ 40 °C et environ 60 °C avant qu'il soit hydrolysé.

3. Amidon hydrolysé par voie enzymatique destiné à être utilisé en tant qu'agent stabilisant comprenant un amidon d'anhydride n-octényl succénique qui a été gélatinisé à une température comprise entre environ 120 °C et 150 °C et hydrolysé par une enzyme ayant une activité endohydrolytique, dans lequel l'enzyme est une alpha fongique-amylase.

4. Emulsion comprenant l'amidon hydrolysé par voie enzymatique selon la revendication 3.

5. Emulsion selon la revendication 4, dans laquelle l'émulsion comprend des gouttelettes d'huile de taille de particules monomodale et à prédominance gaussienne inférieure à environ 5 micromètres, et dans laquelle la taille moyenne de particule est maintenue à moins de 10 % de sa valeur initiale pendant au moins 90 jours lorsqu'elle est conservée à des températures en dessous de 50 °C.

6. Aliment, boisson ou produit industriel comprenant l'amidon hydrolysé de manière enzymatique selon la revendication 3 ou l'émulsion selon les revendications 4 ou 5.
